# EUROPEAN PATENT APPLICATION

(11) **EP 2 088 148 A2**
(43) Date of publication of application: **12.08.2009**
(21) Application number: 09151460.4
(22) Date of filing: 27.01.2009
(51) Int. Cl.: C07D 453/02, A61K 31/435, A61P 13/02

(54) **"Process for the preparation of solifenacin"**

(30) Priority: 08.02.2008 IT MI20080195
(71) Applicant: Dipharma Francis S.r.l., 20021 Baranzate (MI) (IT)
(72) Inventor: Allegrini, Pietro, 20097, S. DONATO MILANESE (MI) (IT); Colombo, Lino, 27100, PAVIA (IT); Brusasca, Marco, 15048, VALENZA PO (AL) (IT); Vladiskovic, Chiara, 20146, MILANO (IT); Razetti, Gabriele, 20099, SESTO SAN GIOVANNI (MI) (IT); Attolino, Emanuele, 74019, PALAGIANO (TA) (IT)
(74) Representative: Minoja, Fabrizio

(57) **Abstract**

A process for the preparation of (1S,3'R)-quinielidin-3'-yl-1-phenyl-3,4-dihydro-1H-isoquinolin-2-carboxylate, namely solifenacin, comprising the reaction of a compound of formula (**IV**) with a compound of formula (**V**), as herein defined, 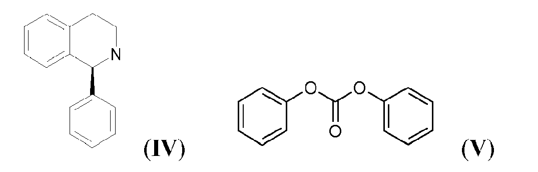 and the subsequent reaction with 3-quinuclidinol.

## Description

### FIELD OF THE INVENTION

The present invention relates to a process for the preparation of solifenacin, namely (1S,3'R)-quiniclidin-3'-yl-1-phenyl-3,4-dihydro-1H-isoquinolin-2-carboxylate.

### TECHNOLOGICAL BACKGROUND

Quiniclidinol derivatives, specific muscarinic antagonists for M₃ receptors, are known from EP 801067, wherein the synthesis of solifenacin according to the following scheme is specifically reported 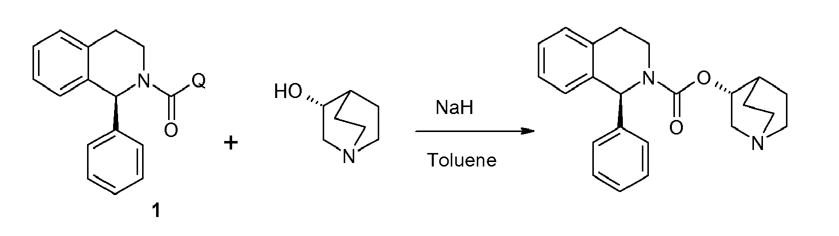 wherein Q is a leaving group, preferably an alkoxide.

Another synthetic route for the preparation of solifenacin is reported in EP 1726304, according to the following scheme: 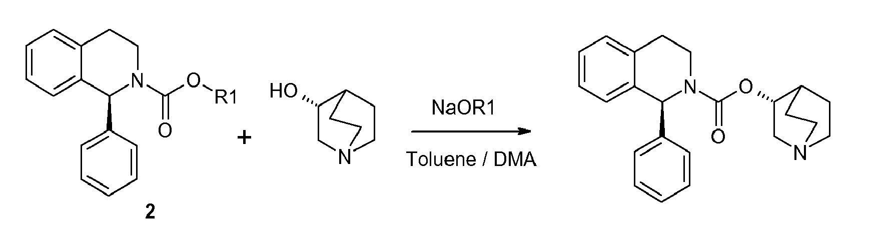 wherein R1 is a lower alkyl.

The preparation of intermediates **1** and **2** according to known methods makes use of chloroformates, which are known to be lacrimogenic, toxic compounds and suspected to be cancerogenic.

Furthermore, it should be noted that the preparation of solifenacin according to EP 1726304 involves formation of the by-product **3**, substituted at the 2- position of the quinuclidinol group 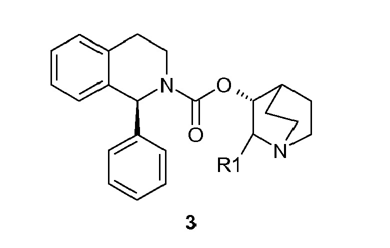 wherein R1 is as defined above.

There is therefore the need for an alternative synthesis which provides solifenacin, which neither uses reactives that are suspected to be cancerogenic nor involves formation of the 2-substituted by-product.

### SUMMARY OF THE INVENTION

It has now been found an alternative process which provides solifenacin while overcoming the above mentioned problems. This makes the process of the invention advantageous over those of the prior art.

### BRIEF DISCLOSURE OF THE ANALYTICAL METHODS

¹H-NMR spectra were recorded on a Varian Mercury 300 spectrometer or Brucker 400 spectrometer, using DMSO-d6 or deuterated chloroform as the solvent.

Particles size was determined with the known laser light scattering technique using a Malvern Mastersizer MS1 instrumentation under the following operative conditions:
- 300RF mm lens with of 2.4 mm laser beam length;
- sample of 500 mg dispersed in 10 ml of hexane (reagent ACS) with 1% SPAN 85^{®}, without presonication, and 2500 rpm stirring rate.

### DETAILED DISCLOSURE OF THE INVENTION

Object of the invention is a process for the preparation of (1S,3'R)-quiniclidin-3'-yl-1-phenyl-3,4-dihydro-1H-isoquinolin-2-carboxylate, or a salt thereof, as a single enantiomer of formula **(I)** or as a mixture of stereoisomers; 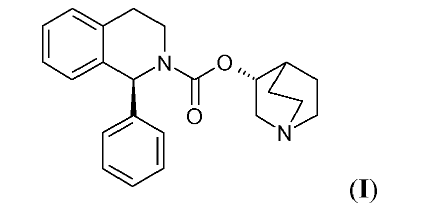 comprising:
the reaction of a compound of formula **(IV),** either as the single (S) enantiomer or as a mixture of enantiomers, 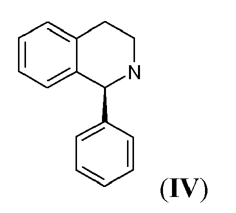
with diphenyl carbonate of formula **(V),** 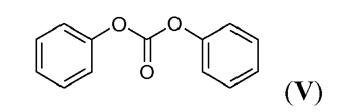
to give a compound of formula **(II),** either as the single (S) enantiomer or as a mixture of enantiomers; and 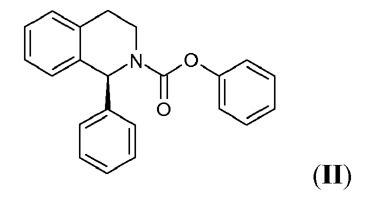
the reaction of a compound **(II)** with 3-quinuclidinol of formula **(III)** as the single enantiomer (R) or as a mixture of enantiomers or a salt thereof, 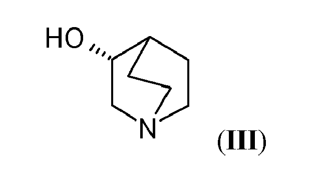
in the presence of a base, to give a compound of formula **(I)** or a salt thereof, as the single enantiomer (1S,3'R) or as mixture of stereoisomers;
and, if the case, the separation of the single enantiomer (1S,3'R) from a mixture of stereoisomers of a compound of formula **(I);** and/or, if desired, the conversion of a compound of formula **(I)** to a salt thereof and/or vice versa.

The reaction between a compound of formula **(IV)** and a compound of formula **(V)** can be carried out in a solvent, typically an organic solvent, selected e.g. from a dipolar aprotic solvent, typically dimethylformamide, dimethylacetamide, acetonitrile, dimethylsulfoxide; an ether, e.g. diethyl ether, methyl-tert-butyl ether, tetrahydrofuran or dioxane; an apolar solvent, such as an aliphatic hydrocarbon, e.g. hexane or cyclohexane, or an aromatic hydrocarbon, e.g. benzene or toluene; or mixtures of two or more, preferably of two or three, of said solvents. The reaction is preferably carried out in tetrahydrofuran or toluene.

The reaction between a compound of formula **(IV)** and a compound of formula **(V)** can be carried out in the presence of a catalyst, e.g. a tertiary amine, typically 4-dimethylaminopyridine.

A resulting compound of formula **(II)** has optical purity approximately equal to or higher than 95%, preferably higher than 98%, more preferably higher than 99%.

The reaction between a compound of formula **(II)** and a compound of formula **(III)** can be carried out in a solvent, typically an organic solvent, selected e.g. from a dipolar aprotic solvent, typically dimethylformamide, dimethylacetamide, acetonitrile, dimethylsulfoxide; an ether, e.g. diethyl ether, methyl-tert-butyl ether, tetrahydrofuran or dioxane; an apolar solvent, such as an aliphatic hydrocarbon, e.g. hexane or cyclohexane, or an aromatic hydrocarbon, e.g. benzene or toluene; or mixtures of two or more, preferably of two or three, of said solvents. The reaction is preferably carried out in toluene or in a toluene-dimethylacetamide mixture.

Preferably at least one of the reactions between a compound of formula **(II)** and a compound of formula **(III)** and between a compound of formula **(IV)** and a compound of formula **(V)** is carried out in an organic solvent.

A base is typically a strong base, such as sodium hydride; a tertiary amine, e.g. diazabicyclooctane or diazabicycloundecene; a carbanion, e.g. butyl lithium or hexyl lithium; an azanion, e.g. lithium diisopropylamide or lithium tetramethylpiperidide, sodium or potassium hexamethyldisilazide; a C₁-C₄ alkoxide, e.g. sodium or potassium methoxide, ethoxide, butoxide or tert-butoxide; preferably sodium hydride.

The reaction between a compound of formula **(II)** and a compound of formula **(III)** can be carried out at a temperature approx. ranging from -15°C to the reflux temperature of the solvent, preferably at about 60°C.

The separation of the single (1S,3'R) enantiomer from a mixture of stereoisomers of formula **(I)** can be carried out according to methods known in the art, for example by fractional crystallization or formation of a diastereomeric salt.

It should be noted that no by-products of formula **(3)** substituted at the 2- position of the quinuclidinol group are formed during the reaction between a compound of formula **(II)** and a compound of formula **(III);** moreover, the process makes no use of chloroformates, with remarkable advantages for the production of solifenacin on large scale and for workers' health.

A compound of formula **(I)** obtained by reaction of a compound of formula **(II)** with optical purity equal to or higher than 95% with 3-(R)-quinuclidinol of similar optical purity, has enantiomeric purity equal to or higher than 95%. Furthermore, said purity can be increased by means of known techniques, for example by crystallization of a diastereomeric salt of addition with a carboxylic or sulfonic acid, such as camphorsulfonic acid, mandelic acid, tartaric acid or dibenzoyltartaric acid.

A compound of formula **(I),** in particular solifenacin, obtained according to the process of the invention, has purity equal to or higher than 95%; preferably equal to or higher than 99%; more preferably equal to or higher than 99.7%.

A single enantiomer (1S,3'R) can be separated from a mixture of stereoisomers of a compound of formula **(I)** according to known methods.

Analogously a compound of formula **(I)** can be converted into a salt thereof or viceversa according to known methods.

A resulting solifenacin salt, in particular solifenacin succinate, has mean particle size D₅₀ approximately ranging from 10 to 250 micrometers, said size can be further reduced by a fine grinding process following known techniques or it can be increased by controlling the crystallization conditions, for example slowly cooling the solution, as it is well known.

The compound of formula **(IV)** is known, and can be prepared, for example, according to the procedure reported in J. Med. Chem., 2005, 48, 6597-6606.

In the present invention, "compound of formula **(I), (III)** and **(IV)"** means the compound as it is or a salt thereof, in particular a pharmaceutically acceptable salt thereof obtained by reaction with an acid selected from those conventionally used; for example sulfate, hydrochloride, succinate, benzenesulfonate, hydrobromide, acetate, formate, propionate, mandelate, tartrate, dibenzoyl tartrate or camphorsulfonate. Preferably the salt of a compound of formula (I) is the succinate.

The compounds of formula **(I), (III)** and **(IV)** can be converted to the salts thereof, and vice versa, according to known methods.

The following examples illustrate the invention.

### Example 1: Preparation of phenyl 1-phenyl-3,4-dihydro-1H-isoquinoline-2-carboxylate (II)

A solution of racemic 1-phenyl-1,2,3,4-tetrahydro-isoquinoline (100 mg, 0.48 mmols) in tetrahydrofuran (500 µl) is added with diphenyl carbonate (102.36 mg, 0.48 mmols) and dimethylaminopyridine in catalytic amount and refluxed. After completion of the reaction, the solvent is evaporated off under reduced pressure and the product is isolated by flash chromatography. The title product is obtained as a yellow oil in 75% yield.

¹⁻H-NMR (400 MHz, CDCl₃) δ 2.85-2.94 (1H, m), 3.10-3.21 (1H, m), 3.35-3.56 (1H, br s), 4.22-4.31 (1H, m), 6.54-6.59 (1H, s), 7.09-7.44 (15H, m).

### Example 2: Preparation of phenyl (S)-1-phenyl-3,4-dihydro-1H-isoquinoline-2-carboxylate (II)

A solution of (S)-1-phenyl-1,2,3,4-tetrahydro-isoquinoline (35 g, 167 mmols) in toluene (175 ml) is added with diphenylcarbonate (35.8 g, 167 mmols) and dimethylaminopyridine in catalytic amount and refluxed. After completion of the reaction, the mixture is left to cool and the toluene solution is washed with 5% NaOH, 1M HCl and brine. The solvent is evaporated off under reduced pressure and the title product is obtained as a yellow oil in 94% yield and 95% optical purity.

¹⁻H-NMR (400 MHz, CDCl₃) ppm δ 2.85-2.94 (1H, m), 3.10-3.21 (1H, m), 3.35-3.56 (1H, br s), 4.22-4.31 (1H, m), 6.54-6.59 (1H, s), 7.09-7.44 (15H, m).

### Example 3: Preparation of quiniclidin-3'-yl-1-phenyl-3,4-dihydro-1H-isoquinolin-2-carboxylate (I)

A solution of racemic phenyl 1-phenyl-3,4-dihydro-1H-isoquinoline-2-carboxylate (88.5 mg, 0.27 mmols) in toluene (770 µl) is added with quinuclidinol hydrochloride (131.8 mg, 0.81 mmols) and NaH (38.8 mg, 1.62 mmols) and refluxed. After completion of the reaction, the mixture is added with brine and extracted with ethyl acetate. The organic phases are washed with water and 20% HCl. The aqueous phases are adjusted to pH 9-10 with 1M NaOH and extracted with ethyl acetate. The organic phases are washed with brine, dried over Na₂SO₄ and concentrated under reduced pressure, thereby obtaining a yellow oil.

¹⁻H-NMR (400 MHz, CDCl₃) ppm δ 1.55-2.18 (5H, m), 2.53-3.16 (10H, m), 3.90-4.20 (1H, m), 6.17-6.55 (1H, m), 7.13-7.42 (9H, m).

### Example 4: Preparation of (1S,3'R)-quiniclidin-3'-yl-1-phenyl-3,4-diidroisoquinoline-2-carboxylate (I)

A solution of sodium hydride (8 g of 60% NaH in mineral oil, 200 mmols) in dimethylacetamide (120 ml) cooled at 0°C is added with (R)-3-quinuclidinol (20.6 g, 162 mmols) in portions. The mixture is then left to warm to room temperature and a solution of phenyl (S)-1-phenyl-3,4-dihydro-isoquinoline-2-carboxylate (43.1 g, 124 mmols) in toluene (180 ml) is added, heated to 60°C and left to react. When the starting material has disappeared, 200 ml of water and 100 ml of toluene are added. The phases are separated, the toluene phase is extracted with 1M HCl. The acidic solution is then stirred with 200 ml of ethyl acetate and adjusted to basic pH. The phases are separated again and the organic phase is concentrated to dryness. The title product is obtained as a yellow oil, with 95% optical purity.

¹⁻H-NMR (400 MHz, CDCl₃) ppm δ 1.55-2.18 (5H, m), 2.53-3.16 (10H, m), 3.90-4.20 (1H, m), 6.17-6.55 (1H, m), 7.13-7.42 (9H, m).

### Example 5: Preparation of (1S,3'R)-quiniclidin-3'-yl-1-phenyl-3,4-diidroisoquinoline-2-carboxylate succinate (I)

A solution of 48 g of solifenacin in 450 ml of ethyl acetate and 90 ml of ethanol is prepared in a 1000 ml four-necked flask equipped with mechanical stirrer, thermometer and condenser, at room temperature. The mixture is refluxed and added with 16 g of succinic acid. The mixture is kept at this temperature for about 10 minutes, then left to spontaneously cool to about 20°C and finally cooled to 0°C on an ice bath. A white solid precipitates which is recovered by filtration. The solid is washed with 2x50 ml of ethyl acetate and subsequently dried in a static dryer, thereby obtaining 53 g of solifenacin succinate with purity equal to 99.7%, optical purity higher than 95%, and mean particle size D₅₀ of approximately 50 micrometers.

## Claims

1. A process for the preparation of (1S,3'R)-quiniclidin-3'-yl-1-phenyl-3,4-dihydro-1H-isoquinolin-2-carboxylate or a salt thereof, either as a single enantiomer of formula **(I)** or as a mixture of stereoisomers; 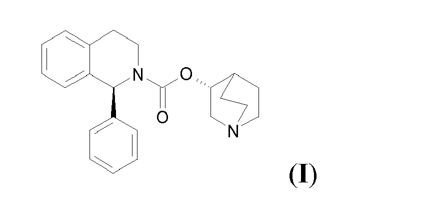 comprising:
the reaction of a compound of formula **(IV),** either as the single (S) enantiomer or as a mixture of enantiomers, 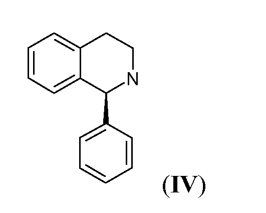
with diphenyl carbonate, of formula **(V),** 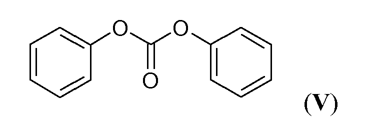
to give a compound of formula **(II),** either as the single (S) enantiomer or as a mixture of enantiomers; and 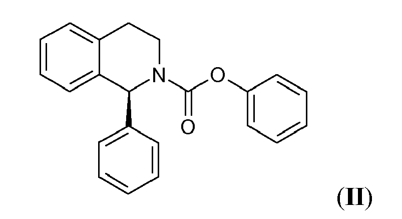
the reaction of a compound **(II)** with 3-quinuclidinol of formula **(III),** either as the single enantiomer (R) or as a mixture of enantiomers, or a salt thereof 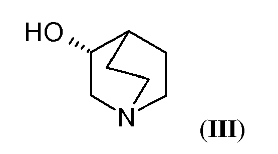
in presence of a base, to give a compound of formula **(I)** or a salt thereof, as the single (1S,3'R) enantiomer or as a mixture of stereoisomers; and, if the case, the separation of the single (1S,3'R) enantiomer from a mixture of stereoisomers of a compound of formula **(I);** and/or, if desired, the conversion of a compound of formula **(I)** to a salt thereof and/or vice versa.

2. The process according to claim 1, wherein the reaction between a compound of formula **(IV)** and a compound of formula **(V)** is carried out in the presence of a catalyst.

3. The process according to claim 2, wherein the catalyst is a tertiary amine.

4. The process according to claim 1, wherein the base is selected from sodium hydride, a tertiary amine, a carbanion, an azanion or a C₁-C₄ alkoxide.

5. The process according to claim 1, wherein at least one of the reactions between a compound of formula **(II)** and a compound of formula **(III)** and between a compound of formula **(IV)** and a compound of formula (V) is carried out in an organic solvent.

6. The process according to claim 5, wherein the solvent is selected from a dipolar aprotic solvent, an ether or an apolar solvent; or mixtures of two or more of said solvents.

7. The process according to claim 6, wherein the reaction between a compound of formula **(II)** and a compound of formula **(III)** is carried out in toluene or in a toluene-dimethylacetamide mixture, and the reaction between a compound of formula **(IV)** and a compound of formula **(V)** is carried out in tetrahydrofuran or toluene.

8. (1S,3'R)-Quiniclidin-3'-yl-1-phenyl-3,4-dihydro-1H-isoquinolin-2-carboxylate with optical purity equal to or higher than 95%.

9. (1S,3'R)-Quiniclidin-3'-yl-1-phenyl-3,4-dihydro-1H-isoquinolin-2-carboxylate with purity equal to or higher than 99.7%.

10. (1S,3'R)-Quiniclidin-3'-yl-1-phenyl-3,4-dihydro-1H-isoquinolin-2-carboxylate succinate with mean particle size D₅₀ ranging from about 10 to about 250 micrometers.
